Europäisches Patentamt

(19) European Patent Office   (11) Numéro de publication:   **0 050 533**

Office européen des brevets   **B1**

(12)   **FASCICULE DE BREVET EUROPEEN**

(45) Date de publication du fascicule du brevet: **27.02.85**   (51) Int. Cl.⁴: **A 61 F 2/36**

(21) Numéro de dépôt: **81401250.6**

(22) Date de dépôt: **31.07.81**

(54) **Prothèse fémorale à tige auto-blocante.**

(30) Priorité: **17.10.80 FR 8022305**

(43) Date de publication de la demande:
**28.04.82 Bulletin 82/17**

(45) Mention de la délivrance du brevet:
**27.02.85 Bulletin 85/09**

(84) Etats contractants désignés:
**AT BE CH DE GB IT LI LU NL SE**

(56) Documents cités:
**DE-A-2 811 603**
**DE-A-2 933 237**
**FR-A-1 046 516**
**FR-A-2 404 429**
**FR-A-2 425 237**
**FR-A-2 438 469**
**US-A-3 996 625**

(73) Titulaire: **Bréard, Francis Henri**
**25 rue de Coulmiers**
**F-75014 Paris (FR)**

(72) Inventeur: **Bréard, Francis Henri**
**25 rue de Coulmiers**
**F-75014 Paris (FR)**

(74) Mandataire: **Lemoine, Robert et al**
**Cabinet Malémont 42, Avenue du Président**
**Wilson**
**F-75116 Paris (FR)**

Courier Press, Leamington Spa, England.

## Description

L'invention a pour objet une prothèse fémorale auto-blocante symétrique par rapport à un plan longitudinal et comprenant une tige fémorale intramédulaire, une platine solidaire de la tige et une tête de forme générale sphérique portée par un col solidaire de la platine, prothèse dont la tige fémorale se prolonge par une partie cylindrique lisse destinée à être montée dans le canal médulaire préalablement évidé selon un diamètre correspondant, l'extrêmité libre de cette partie lisse allant en se rétréissant et étant fondue longitudinalement de façon à fourer sur bec dont les mâchoires frevent de rapprocher. Une prothése de ce type est décrite dans le brevet US—A—3996625. On connaît le succès de ces prothèses qui tendent à remplacer les prothèses classiques qui obligeaient à sceller la tige fémorale à l'aide d'un ciment acrylique traumatisant pour le malade en cas d'ablation, et souvent à l'origine de réactions tissulaires, ou celles à auto-blocage par pontage osseux à travers une fenêtre ou par frittage, mais qui nécessitaient une période relativement longue d'alitement tout en restant douloureuses à cause des défectuosités de la fixation.

Contrairement à ces prothèses classiques, les prothèses fémorales à tige cylindrique lisse ont le grand avantage d'être auto-blocantes dans leur logement, sans adjonction de ciment et sans pontage osseux, et de permettre une ablation aissée, le dégagement de la tige du fémur étant extrêmement facile.

Elles présentent cependant un certain nombre d'inconvénients liés aux exigences qu'implique l'utilisation d'une tige cylindrique lisse. En particulier, la réalisation d'un logement dans le fémur exige non seulement d'enlever l'os spongieux du canal intramédulaire du fémur sur une longueur correspondant à la longueuer de la tige, mais encore de fraiser le fémur de façon à obtenir un diamètre correspondant exactement au diamètre de la partie cylindrique de la tige. Or, il est bien connu que le fémur présente, essentiellement dans le plan transversal par rapport à la prothèse, une courbure telle que ce logement cylindrique va entamer la partie osseuse dure jusqu'au voisinage de la zone extérieure de l'os, en particulier au niveau de l'extrémité inférieure de la tige. C'est d'ailleurs pourquoi, en pratique, il faut se résoudre à donner à la tige des prothèses une certaine courbure dans ce même plan, ce qui contraint à disposer d'une réserve de prothèses pour la jambe gauche et d'une autre réserve pour la jambe droite, chaque fois bien entendu avec tous les diamètres conventionnels. Un tel stock permet de donner au logement de la prothèse une certaine courbure qui lui permet de suivre l'axe du fémur et donc évite que le logement de la tige n'affleure par trop les zones extérieures de l'os.

L'invention a pour objet de remédier à ces inconvénients et de mettre à la disposition des chirurgiens une prothèse apte à être utilisée aussi bien pour la jambe gauche que pour la jambe droite dans un logement qui peut être fraisé selon une légère courbure et qui donc sauvegarde la solidité de l'os.

Pour ce faire, elle vise une prothèse qui ce caractérise essentiellement en ce que l'extrémité libre de la partie cylindrique lisse de la tige est conformée en ogive et en ce qu'au moins une fente est située dans le plan de symétrie de la prothèse.

Grâce à cette disposition, l'une des mâchoires au moins peut, en se rapprochant élastiquement de l'autre, épouser la forme du logement, même si ce dernier est incurvé dans sa partie terminale.

En outre, la fente participe au blocage en rotation de la prothèse par macroprolifération osseuse.

Enfin, la présence d'une des mâchoires du bec, de partet d'autre du plan longitudinal de symétrie de la prothèse, permet d'utiliser le même appareil soit pour la jambe gauche, soit pour la jambe droite.

Il est à noter que la conformation en ogive de l'extrémité de la tige aide la pénétration et confère une élasticité croissante aux deux mâchoires du bec.

Si l'on considère que l'élasticité à conférer à l'extrémité inférieure de la partie cylindrique de la tige doit être plus grande, il est possible de prévoir, conformément à l'invention, une seconde fente axiale perpendiculaire à la première.

Avantageusement, une rainure prolonge latéralement l'une des fentes sur la plus grande portion de la tige. Cette rainure évite l'effet de piston pendant l'intervention chirurgicale et participera ensuite à l'ancrage antirotation.

Un mode d'exécution de la présente invention est décrit ci-après en référence au dessin annexé dans lequel:

La figure 1 est une vue de profil d'une prothèse conforme à l'invention, en place;

Les figures 2 et 3 sont des vues en coupe de la prothèse selon les lignes II—II et III—III de la figure 1;

La figure 4 montre une variante de l'invention; et

La figure 5 est une vue de face de la même prothèse, en place.

La prothèse fémorale représentée sur les figures comprend un plan de symétrie longitudinal, une tige fémorale intramédulaire à blocage diaphysaire désignée dans son ensemble par 1, une platine 2 à appui cortical solidaire de la tige et une tête 3 de forme générale sphérique portée par un col 4 solidaire de la tige 1 par l'intermédiaire de la platine 2.

La tige fémorale 1 est prolongée par une partie sensiblement cylindrique 5 à diamètre égal au diamètre du logement médulaire du fémur dans lequel la tige doit être implantée.

3    **0 050 533**    4

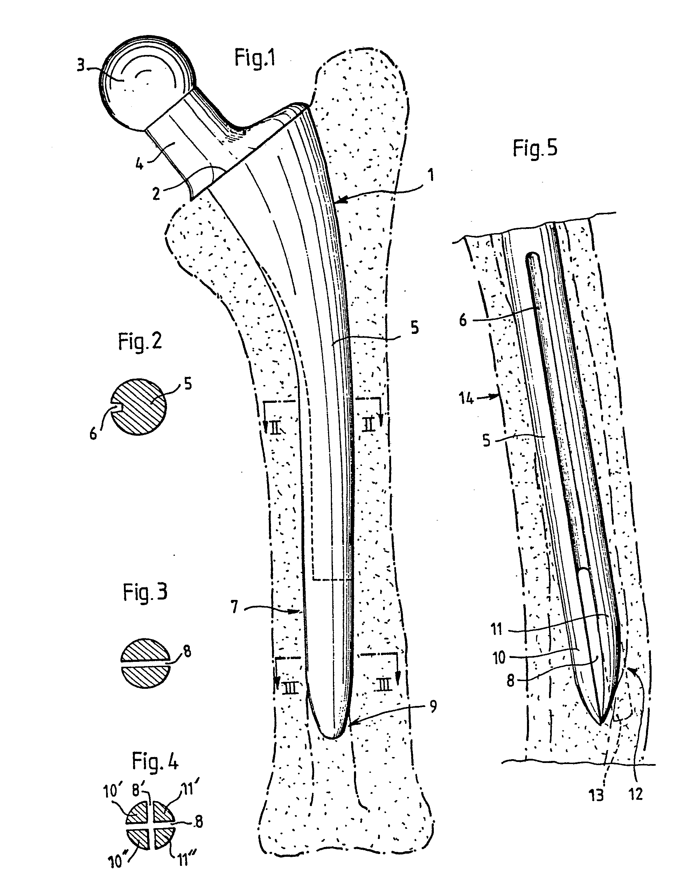

Une rainure longitudinale 6 est prévue en outre sur la face extérieure de la tige fémorale, rainure qui s'étend sur toute la longueur du prolongement cylindrique 5 et sur une partie adjacente à ce dernier, comme visible sur la figure 1.

Conformément à l'invention, l'extrémité inférieure 7 de la partie cylindrique 5 de la tige est pourvue d'une fente longitudinale 8 disposée dans le plan de symétrie de la prothèse qui est d'une largeur qui peut, par exemple, être de l'ordre du 5ème du diamètre de la tige.

En outre, l'extrémité de la tige est conformée comme on le voit en 9 en forme d'ogive pour se terminer en pointe et constituer ainsi, avec la fente 8, deux mâchoires 10 et 11.

La figure 5, qui est une vue de face partielle de la prothèse en place dans le fémur, montre la courbure de cet os dans le plan transversal par rapport à la prothèse. On conçoit que si la tige 5 était entièrement cylindrique, son logement occuperait, dans la zone 12, un espace représenté en pointillés avec la référence 13 et affleurerait donc la surface de l'os le rendant particulièrement fragile dans cette zone.

Naturellement, il aurait été possible de faire occuper au logement une position moins inclinée pour qu'il se trouve axé au niveau de la zone 12, mais c'est alors dans la zone 14 que le logement aurait entamé dangereusement la partie osseuse.

C'est pourquoi les chirurgiens ont été amenés à donner au logement, et donc à la tige, un légère courbure avec les inconvénients précisés plus haut.

Ces inconvénients sont entièrement éliminés grâce au fait que la mâchoire 11 s'est déformée élastiquement, guidée par sa pointe le long de la paroi osseuse et s'est rapprochée de la mâchoire 10 sans qu'il ait été nécessaire d'entamer l'os dans la zone 12 et sans qu'il ait été nécessaire de donner la moindre courbure au logement de la tige 5.

Les spécialistes auront reconnu que les figures représentent une prothèse placée sur une jambe gauche. Il est évident que la même prothèse utilisée pour une jambe droite aurait rendu les mêmes services. C'est alors la mâchoire 10 qui se serait déformée pour se rapprocher de la mâchoire 11.

La figure 4 montre une variante qui est une coupe, prise elle aussi dans le plan III—III de la figure 1, dans laquelle on retrouve la fente 8, mais dans laquelle les mâchoires 10 et 11 sont partagées en deux demi-mâchoires 10', 10" et 11', 11" par un fente 8' semblable à la fente 8 pratiquée dans un plan perpendiculaire.

Une telle construction peut être southaitable si l'on désire donner aux mâchoires une plus grande élasticité.

Il va de soi par ailleurs que la prothèse représentée peut s'utiliser aussi bien pour le remplacement partiel de l'articulation de la hanche que pour le remplacement total de celle-ci.

## Revendications

1. Prothèse fémorale auto-blocante symétrique par rapport à un plan longitudinal et comprenant une tige fémorale intramédulaire (1), une platine (2) solidaire de la tige (1) et une tête (3) de forme générale sphérique portée par un col (4) solidaire de la platine (2), prothèse dont la tige fémorale (1) se prolonge par une partie cylindrique lisse (5) destinée à être montée dans le canal intramédulaire du fémur préalablement évidé selon un diamètre correspondant, l'extrémité libre (7) de cette partie cylindrique lisse (5) allant en se rétrécissant et présentant au moins une fente longitudinale (8) d'une certaine largeur, de façon à constituer un bec dont les mâchoires (10, 11) légèrement élastiques peuvent se rapprocher l'une de l'autre, caractérisée en ce que l'extrémité libre (7) de la partie cylindrique lisse (5) de la tige est conformée en ogive (9) et en ce qu'au moins une fente (8) est située dans le plan longitudinal de symétrie de la prothèse.

2. Prothèse selon la revendication 1, caractérisée en ce que l'extrémité inférieure (7) de la partie cylindrique lisse (5) de la tige (1) est pourvue d'une seconde fente axiale (8') perpendiculaire à la première (8).

3. Prothèse selon la revendication 1 ou 2, caractérisée en ce qu'une rainure longitudinale (6) prolonge latéralement l'une des fentes (8, 8') sur la plus grande portion de la tige (1).

## Patentansprüche

1. Selbstsperrende, zu einer Längsebene symmetrische Oberschenkelprothese, umfassend einen intramedullären Oberschenkelschaft (1), eine mit dem Schaft (1) verbundene Platine (2) und einen Kopf (3) von allgemein kugeliger Form, getragen von einem mit der Platine (2) verbundenen Hals (4), deren Oberschenkelschaft (1) durch einen zylindrischen, glatten Teil (5) verlängert ist, dazu bestimmt, im zuvor gemäß einem entsprechenden Durchmesser freigemachten Markkanal des Oberschenkelknochens montiert zu werden, wobei das freie Ende (7) dieses zylindrischen, glatten teils (5) sich verjüngend ausläuft und wenigstens einen Längsspalt (8) bestimmter Breite aufweist, um einen Schnabel zu bilden, dessen leight elastische Backen (10, 11) sich einander nähern können, dadurch gekennzeichnet, daß das freie Ende (7) des zylindrischen, glatten Teils (5) des Schaftes bogenförmig (9) ist und daß wenigstens ein Spalt (8) in der Symmetrie-Längsebene der Prothese liegt.

2. Prothese nach Anspruch 1, dadurch gekennzeichnet, daß das untere Ende (7) des zylindrischen, glatten Teils (5) des Schaftes (1) mit einem zweiten, axialen Spalt (8') senkrecht zum ersteren (8) versehen ist.

3. Prothese nach Anspruch 1 oder 2, dadurch gekennzeichnet, daß eine Längsnut (6) eine der Spalten (8, 8') über den größten Teil des Schaftes (1) seitlich verlängert.

## Claims

1. A femoral self-locking prosthesis symmetrical with respect to a longitudinal plane and comprising a femoral intramedullar rod (1), a plate (2) integral with the rod (1) and a generally spherical shaped head (3) supported by a neck (4) integral with the plate (2), in which prosthesis the femoral rod (1) is extended by a smooth cylindrical part (5) intended to be mounted in the intramedullar canal of the femur previously hollowed out to a corresponding diameter, the free end (7) of this smooth cylindrical part (5) being progressively tapered and having at least one longitudinal slit (8) of a certain width so as to form a nose whose slightly resilient jaws (10, 11) may draw near to one another, characterized in that the free end (7) of the smooth cylindrical part (5) of the rod is given an ogival shape (9) and in that at least one slit (8) is situated in the longitudinal plane of symmetry of the prosthesis.

2. Prosthesis according to claim 1, characterized in that the lower end (7) of the smooth cylindrical part (5) of the rod (1) is provided with a second axial slit (8') perpendicular to the first one (8).

3. The prosthesis according to claim 1 or 2, characterized in that a longitudinal groove (6) extends one of the slits (8, 8') laterally over the greatest portion of the rod (1).

Fig.1

Fig.2

Fig.3

Fig.4

Fig.5